# EUROPEAN PATENT APPLICATION

(11) **EP 0 825 438 A2**
(43) Date of publication of application: **25.02.1998**
(21) Application number: 97660084.1
(22) Date of filing: 22.07.1997
(51) Int. Cl.: G01N 33/38, G01N 33/24, G01N 3/40, G01B 7/06

(54) **Method and apparatus for measuring the compression strength, elasticity, hardness, thickness or the like property of a moving web, such as a mineral wool web**

(30) Priority: 20.08.1996 FI 963249
(71) Applicant: Isover Saint-Gobain, 92096 La Défense Cedex (FR)
(72) Inventor: Lehtinen, Mikko, 05830 Hyvinkää (FI); Salo, Matti, 31300 Tammela (FI)
(74) Representative: Blomquist, Tuula Maria Ingeborg

(57) **Abstract**

A method and an apparatus for measuring the compression strength, elasticity, hardness, thickness or the like property of a moving web, such as a mineral wool web, moving on a supporting base. The measuring is carried out by means of a measuring unit (10) including a pressure member or measuring head (76, 77), disposed above the web, which measuring unit during the measurement is moved at the same velocity as the web in the direction of the motion of the web. The actual measuring is carried out by pressing the pressure member or measuring head (76, 77) during the measurement against the moving web so that the web is compressed against the base (14) supporting it.

## Description

The present invention relates to a method and an apparatus for measuring the compression strength, elasticity, hardness, thickness or the like property of a moving web, such as a mineral wool web, moving on a supporting base, as defined in the first part of the claims 1 and 8.

The above mentioned properties are vital for the mineral wools as well as for many other sheet-like products used in the building industry for sound and heat insulating purposes. Such other sheet-like products are for instance sheets made of polystyrene an polyurethane.

In connection with the production of the sheets, the compression strength, elasticity, hardness, thickness or other such properties have earlier been monitored by taking samples of the product and studying them to determine these properties. It is evident that it takes time to take samples and study them, which means that the changes in the production process are only made with a relatively great delay.

The principal object of the present invention is to provide a method and apparatus by means of which the above mentioned drawbacks can be minimized.

It is a special object of the present invention to provide a method and apparatus by means of which it is possible to measure the compression strength, elasticity, hardness, thickness or the like property of a sheet-like product on-line, in other words, directly from the web at production, and without breaking the product.

To achieve these objects, the method and apparatus according to the invention is characterized by what is defined in the characterizing parts of claims 1 and 8.

According to a method of the invention, the measuring of the compression strength, elasticity, hardness, thickness or the like property of a moving web, such as a mineral wool web, moving on a supporting base, is carried out by means of a measuring unit disposed above the web, which during the measurement is moved at the same velocity as the web in the direction of the motion of the web. A pressure member or measuring head in the measuring unit is pressed during the measurement against the moving web so that the web is compressed against the base supporting it. The measuring completed, the pressure member of the measuring unit is lifted off the web, the moving of the measuring unit along the web is stopped and the measuring unit returned to its initial position for new measurement by moving it back in the longitudinal direction of the web. The measurements may be performed at regular intervals and/or when necessary. The transversal measuring point can be chosen by transferring the measuring unit before the measuring to the correct point in the transversal direction of the web.

The apparatus according to a preferred embodiment of the invention thus comprises, apart from the measuring unit, also means for transferring the unit both in the longitudinal and the transversal direction of the web. These means comprise:
- longitudinal guide bars, along which the measuring unit can be transferred in the direction of the motion of the web during the measurement, the length of the guide bars being e.g. 3000 - 1000 mm, preferably about 2200 mm, the length of movement of the measuring unit on the guide bars being e.g. 2000 - 1000 mm, preferably about 1600 mm;
- means, such as a compressed-air cylinder, for returning the measuring unit after the measurement against the direction of motion of the web to its initial position;
- transversal guide bars along which the measuring unit can be moved to the measuring point in the transverse direction of the web, the length of the transversal guide bars being dependent of the width of the web;
- means, such as a compressed-air cylinder, for moving the measuring unit in the transverse direction of the web before and after the measurement; and
- gripping members or the like for attaching the measuring unit to the moving web, preferably to its upper surface, for the duration of the measurement.

According to a preferred embodiment of the invention, the measuring of the compression strength or the like property is performed in the following manner.

Prior to beginning the actual measuring, the measuring unit is transferred to the measuring location by e.g. a compressed-air cylinder along the transversal guide bars in the transversal direction of the web.

When the measuring unit has been positioned correctly, it is, for the duration of the measuring, attached to the upper surface of the web moving underneath the measuring unit by means of gripping members disposed in the measuring unit, which gripping members or the like protrude downwards from the gripping member as far as the length of adjustment. Thus the measuring unit is caused to move along the longitudinal guide bars at the same velocity as the web and in the direction of the motion of the web. The velocity of movement of the measuring unit is then 1 - 15 m/min, typically 4 - 6 m/min.

According to a preferred embodiment of the invention, the measuring is carried out by first pressing the pressure member in the measuring unit or its pressure plate down against the upper surface of the web and further downwards toward the web for the length of a given vertical movement, or from the upper surface of the web toward the base supporting the web. The speed of movement of the pressure member is typically 50 - 200 mm/min. In other words, a depression of a certain depth is pressed in the web by means of the pressure member, which depression disappears when the pressing is stopped. The compressive force required to cause the given length of movement or the measuring length, the depression, is proportional to the compression strength, elasticity, hardness, thickness and/or the like property of the web. The compressive force can be measured by e.g. a load cell.

The entire length of the movement of the pressure member of the measuring unit, from the initial to the final position of the pressure member, in other words, until the bottom of the depression, is e.g. 200 - 400 mm, preferably 300 mm. The entire length of the movement depends on e.g. the distance between the measuring unit and the web. The actual measuring length or the predetermined length of movement is shorter than that by the distance between the pressure member and the web, preferably e.g. 250 mm. Even very small measuring lengths, of a few millimetres, are possible to carry out. Typically, the thinnest thickness to be measured is about 15 mm. The accuracy of the measurement is as good as 0.5 mm, even 0.1 mm.

According to another preferred embodiment of the invention, the measuring is carried out by pressing the pressure member with a given pressure against the web, in other words, pressing it against the upper surface of the web and towards the base supporting the web, in which case the length of movement of the pressure member or the measuring head, the measuring length, or the compression of the web, is proportional to the compression strength, elasticity, hardness, thickness and/or the like property of the web. In this case the length of movement caused by a given pressure is being measured. The length of movement of the pressure member or the measuring length can be measured by means of e.g. a linear detector.

After the actual measuring, the measuring unit is detached from the web and returned to its initial position by e.g. a compressed-air cylinder.

Exemplary embodiments of the invention will now be described more in detail with reference to the accompanying drawing, on which
FIG. 1 is a schematic side view of a measuring unit according to the invention arranged above a moving mineral wool web;
FIG. 2 is a schematic cross-sectional view of the measuring unit according to FIG. 1 taken on line AA;
FOG. 3 is a top plan view of the measuring unit according to FIG. 1;
FIG. 4 is a schematic side view of the measuring unit according to FIG. 1 without the supporting frame and guide bars;
FIG. 5 enlarged view of the detail X of FIG. 1, as seen from behind the front gripping member;
FIG. 6 is an enlarged view of the pressure member according to an embodiment of the invention; and
FIG. 7 is an enlarged view of the pressure member according to another embodiment of the invention.

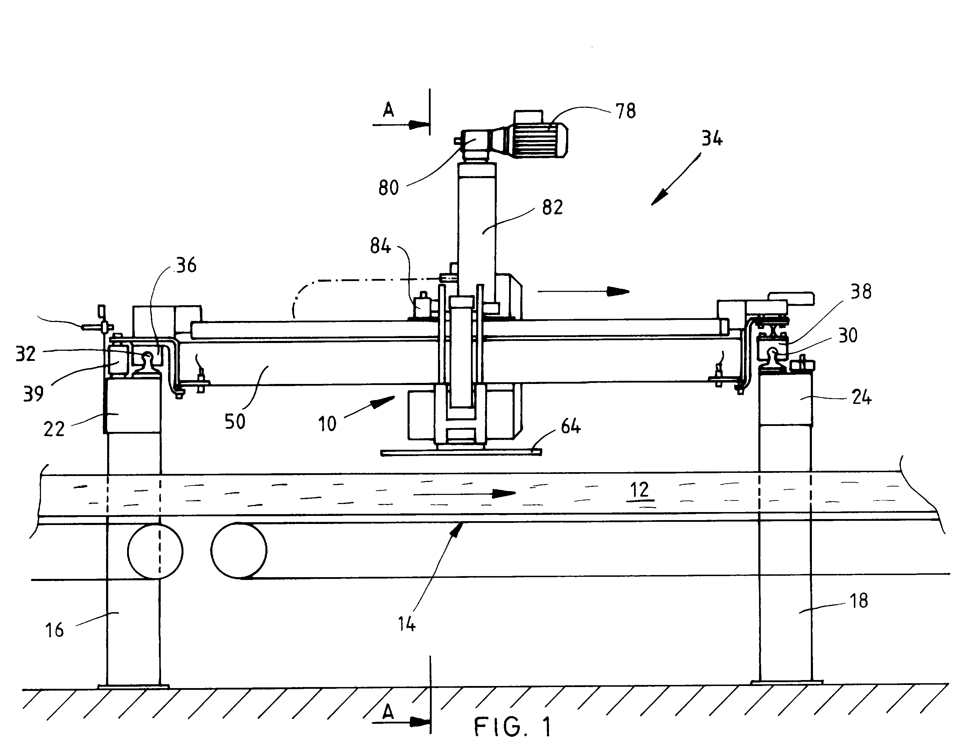

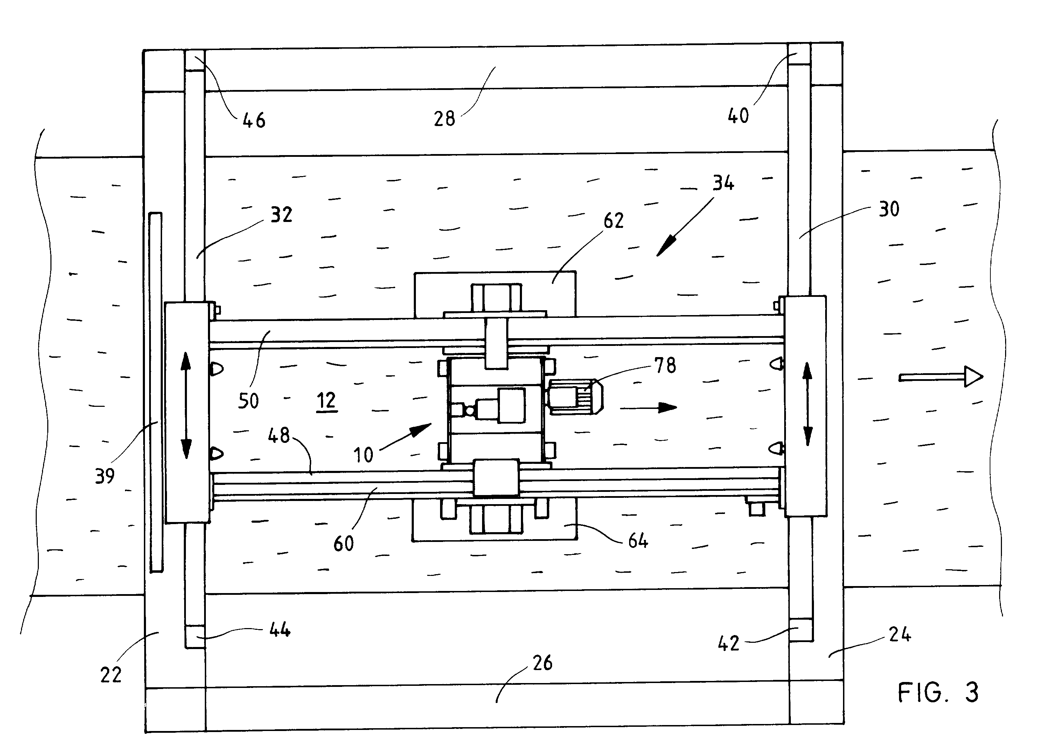

FIGS 1, 2 and 3 show a measuring unit 10 according to the invention disposed on a mineral wool web 12 moving in the direction of the arrow. The mineral wool web is arranged to move on a flat conveyor or base (preferably ± 0.1 mm) 14 below the measuring unit.

The measuring unit 10 is supported by a frame, of which FIGS 1 - 3 show vertical columns 16, 18 and 20, and horizontal transversal beams 22 and 24 and longitudinal beams 26 and 28. Guide bars 30 and 32 are disposed on the transversal beams 22 and 24 by which a measuring carriage 34 supporting the measuring unit 10 travels across the web 12, supported by e.g. ball bushings 36 and 38 and moved by means of a compressed-air cylinder 39. Stoppers 40, 42, 44 and 46 are disposed at the ends of the guide bars 30 and 32, the purpose of which is to prevent the measuring unit from running off the guide bars.

The measuring carriage 34 comprises rails 48 and 50 disposed on both sides of the measuring unit 10 and supporting it, on which rails guide bars 52 and 54 are mounted, along which the measuring unit 10 can easily be moved in the longitudinal direction of the web by means of ball bushings 56 and 58, or the like members. The guide bars and ball bushings make it possible, by means of the web 12, to move the measuring unit in the direction of the motion of the web. The ends of the rails 48 and 50 and those of the guide bars 52 and 54 are supported on the transversal beams 22 and 24 and they travel along the beams across the web 12 by means of the guide bars 30 and 32 and the ball bushings 36 and 38. After the measuring, the measuring unit 10 is returned to its initial position by means of a compressed-air cylinder 60 disposed on the beam 22.

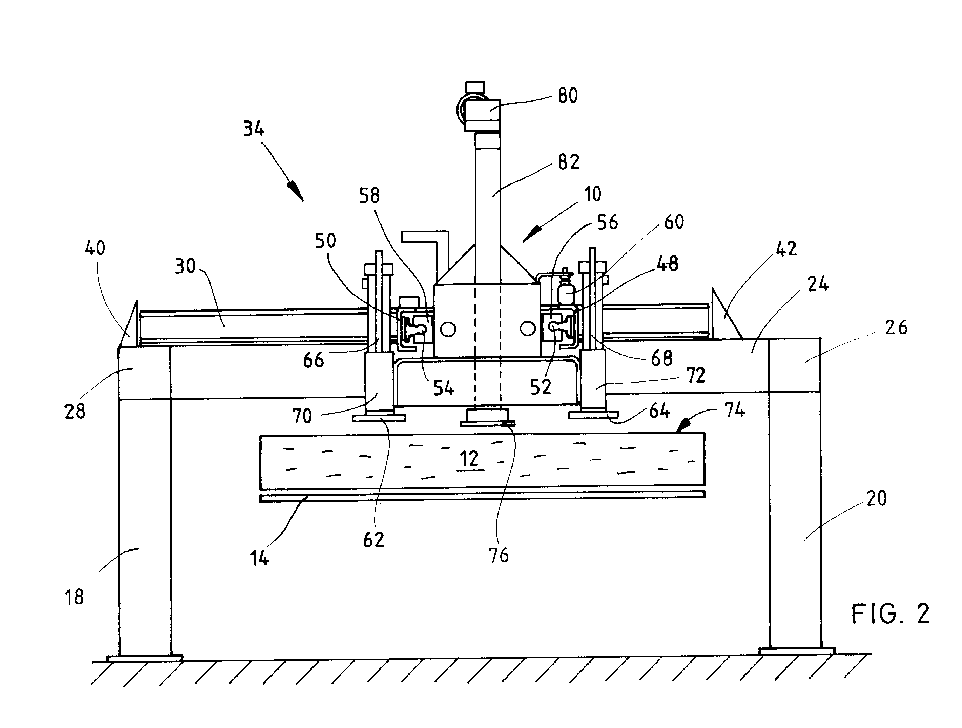

As shown in FIG 2, two gripping members 62 and 64 are connected to the measuring unit 10, which by means of compressed-air cylinders 66 and 68 and guided by guide bars 70 and 72, can be pressed downwards toward the upper surface 74 of the mineral wool web in the way that the measuring unit 10 sticks to the web and travels along with it in the direction of the motion of the web.

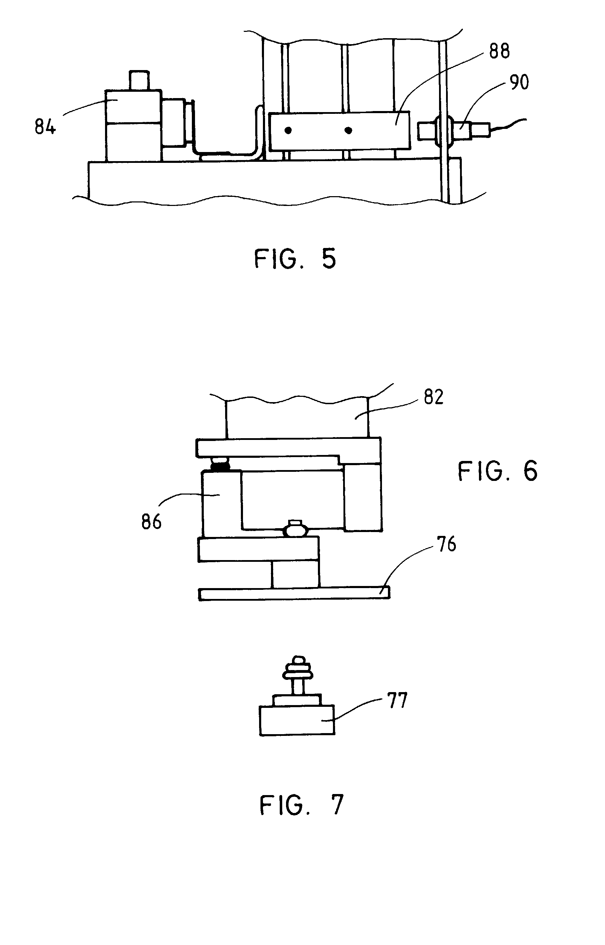

The actual measuring head, or pressure member, 76 is disposed substantially in the middle section of the measuring unit 10, from where it can be pressed downwards toward the mineral wool web 12 by means of a motor 78, a worm gear 80 and a linear unit 82. The actual pressure member is preferably formed of a flat plate; a quadratic plate such as shown in FIG 6, or a round, thicker measuring head 77 with a thickness of e.g. 20 mm, as shown in FIG 7. The area of a quadratic plate is preferably about 200 x 200 mm. The diameter of a round measuring head is e.g. 70 - 90 mm, preferably about 80 mm. A round measuring head can preferably be used for measuring the hardness of webs of higher grades of strength. Different pressure members can be used in the same measuring unit, according to various needs, as far as the compression strength or other properties of the web are concerned.

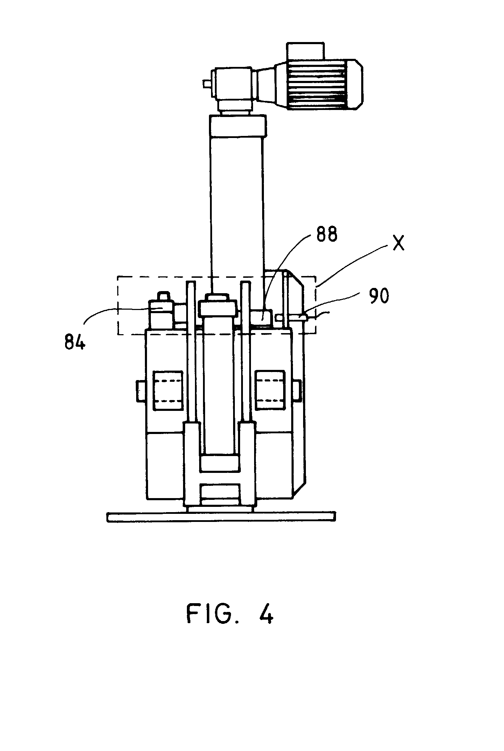

The pressure by which the pressure member 76 is pressed can be preset and the length of the movement or the measuring distance measured by means of e.g. a distance gauge 84 illustrated in FIGS 1, 4 and 5. On the other hand, the pressure member 76 can be pressed from the upper surface 74 downwards toward the base 14 for a given length of movement, in this case measuring the required pressure e.g. by a load cell 86, best shown in FIG 6. The measuring distance can be preset to the desired length by a limit stopper 88 and a limit switch 90, shown in FIGS 4 and 5.

The compression strength of mineral wool webs of various strength grades, such as those of 8 kN/m², 12 kN/m², 20 kN/m² or 25 kN/m², can preferably be measured using a pressure plate of 200 x 200 mm. The compression strength of a higher strength grade, that of 45 kN/m², can preferably be measured using a round measuring head 77, with a diameter of 79.8 mm. The invention is especially useful when the mineral wool to be measured is 250 mm at its maximum and 15 mm at its minimum. The maximum load for strength grade 25 kN/m² is about 1000 N.

The invention renders possible the on-line measurement of the compression strength and other such properties to be measured, without breaking the product, which makes it possible to speedily correct the product quality in the desired direction.

The invention is not limited to the embodiments described and illustrated above, but can be varied in many ways within the scope and spirit of the invention, which is defined in the appended claims.

## Claims

1. A method of measuring the compression strength, elasticity, hardness, thickness or the like property of a moving web (12), such as a mineral wool web, moving on a supporting base, comprising
- carrying out the measurement by means of a measuring unit (10) disposed above the web, which during the measurement is moved at the same velocity as the web in the direction of the motion of the web; and
- pressing the pressure member or measuring head (76, 77) in the measuring unit during the measurement against the moving web so that the web is compressed against the base (14) supporting it.

2. A method according to claim 1, **characterized** in that the measuring unit (10) running on guide bars (52, 54) parallel with the direction of the motion of the web is moved in the direction of the motion of the web by attaching the measuring unit to the web (12) for the duration of the measurement by means of gripping members (62, 64) or the like.

3. A method according to claim 1, **characterized** in that, prior to the beginning of the measurement, the measuring unit (10) is moved to the transversal point of measuring along guide bars (30, 32) running across the web in the transverse direction of the web.

4. A method according to claim 1, **characterized** in that the measurement is carried out by pressing the pressure member (76, 78) against the upper surface (74) of the web so as to effect a given vertical movement of said member towards the base (14) supporting the web, the compressive force required therefore being proportional to the compression strength, elasticity, hardness, thickness and/or the like property of the web.

5. A method according to claim 4, **characterized** in that the compressive force required is recorded by means of a load cell (86).

6. A method according to claim 1, **characterized** in that the measurement is carried out by pressing the pressure member (76, 78) with a given pressure against the upper surface (74) of the web towards the base (14) supporting the web, the length of the movement of the pressure member or the measuring head or the compression of the web being proportional to the compression strength, elasticity, hardness, thickness and/or the like property of the web.

7. A method according to claim 6, **characterized** in that the movement of the pressure member is measured by a linear detector disposed in the measuring unit.

8. An apparatus for measuring the compression strength, elasticity, hardness, thickness or the like property of a moving web, such as a mineral wool web, moving on a supporting base, the apparatus comprising a measuring unit (10) disposed above the web, which unit comprises means for moving the measuring unit at the same velocity as the web in the direction of the web during the measurement, and a pressure member or measuring head (76, 77) which can be pressed against the moving web (12) during the measurement so that the web is compressed against the base (14) supporting it.

9. An apparatus according to claim 8, **characterized** in that
- longitudinal guide bars (52, 54) along which the measuring unit can be moved in the direction of the motion of the web during the measurement;
- means, such as a compressed-air cylinder (60), for returning the measuring unit after the measurement against the direction of motion of the web to its initial position;
- transversal guide bars (30, 32) along which the measuring unit can be moved to the measuring point in the transverse direction of the web;
- means, such as a compressed-air cylinder (39), for moving the measuring unit in the transverse direction of the web before and after the measurement; and
- gripping members (62, 64) or the like for attaching the measuring unit to the moving web for the duration of the measurement.

10. An apparatus according to claim 8, **characterized** in that the measuring unit comprises
- means (76, 78, 80, 82) for pressing the pressure member against the upper surface of the web so as to a effect a given vertical movement of said member towards the base supporting the web; and
- means, such as a load cell (86), for measuring or recording the pressure or compressive force needed to bring about said given movement.

11. An apparatus according to claim 8, **characterized** in that the measuring unit comprises
means (76, 78, 80, 82) for pressing the pressure member with a given pressure or compressive force against the upper surface of the web towards the base supporting the web; and
- means, such as a linear detector (84), for measuring or recording the length of movement of the pressure member brought about by said given pressure or compressive force.
